# EUROPÄISCHE PATENTSCHRIFT

(11) **EP 0 537 535 B1**
(45) Veröffentlichungstag und Bekanntmachung des Hinweises auf die Patenterteilung: **23.07.1997**
(21) Anmeldenummer: 92116523.9
(22) Anmeldetag: 28.09.1992
(51) Int. Cl.: C07C 311/46, C07C 311/41, C07D 211/58

(54) **Sulfonamide, die sich von 1-Hydroxy-6-aminonaphtalin-3-sulfonsäure (I-Säure) ableiten**
Sulfonamides derived from 1-hydroxy-6-aminonaphthaline-3-sulfonic acid
Amides sulfoniques dérivées d'acide naphthalène-3-sulfonique-1-hydroxy-6-amino

(30) Priorität: 10.10.1991 DE 4133514
(43) Veröffentlichungstag der Anmeldung: 21.04.1993
(73) Patentinhaber: BASF Aktiengesellschaft, 67063 Ludwigshafen (DE)
(72) Erfinder: Mayer, Udo, Dr., W-6710 Frankenthal (DE); Schloesser, Ulrike, Dr., W-6800 Mannheim 81 (DE)

(56) Entgegenhaltungen:
- EP-A- 0 594 037
- GB-A- 651 917
- US-A- 4 001 204
- Keine einschl gigen Dokumente gefunden

## Beschreibung

Die vorliegende Erfindung betrifft neue Sulfonamide der Formel I 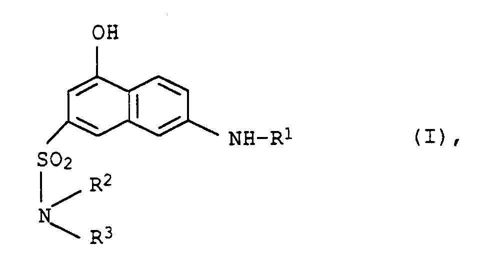 in der
- R¹: C₁-C₁₃-Alkyl, gegebenenfalls substituiertes Phenyl, C₁-C₈-Alkanoyl oder gegebenenfalls substituiertes Benzoyl und
- R² und R³: gleich oder verschieden sind und unabhängig voneinander jeweils C₁-C₁₃-Alkyl, das gegebenenfalls durch 1 bis 4 Imino- oder C₁-C₄-Alkyliminogruppen unterbrochen ist und durch Amino, einen 5- oder 6-gliedrigen heterocyclischen Rest, der ein Stickstoffatom und gegebenenfalls ein weiteres Heteroatom, ausgewählt aus der Gruppe, bestehend aus Stickstoff, Sauerstoff und Schwefel, aufweist, oder gegebenenfalls substituiertes C₅-C₇-Cycloalkyl substituiert sein kann, gegebenenfalls substituiertes C₅-C₇-Cycloalkyl oder gegebenenfalls durch Methyl substituiertes Piperidinyl oder R² auch Wasserstoff bedeuten.

Aus J. Prakt. Chem. 101, 55, 1921, ist das N-Phenylsulfonamid der N-Acetyl- oder N-Benzoyl-I-Säure bekannt.

Weiterhin beschreibt die GB-A-651 917 Azofarbstoffe mit N-Acetyl- oder N-Benzoyl-I-Saure. Schließlich sind in der US-A-4 001 204 Derivate von 1-Hydroxynaphthalin-2-carbonsäreamiden oder -2-sulfonsäureamiden beschrieben.

Aufgabe der vorliegenden Erfindung war es nun, neue Sulfonamide, die sich von 1-Hydroxy-6-aminonaphthalin-3-sulfonsäure (I-Säure) ableiten, bereitzustellen. Die neuen Sulfonamide sollten sich in vorteilhafter Weise zur Herstellung von Farbstoffen eignen.

Demgemäß wurden die eingangs näher bezeichneten Sulfonamide der Formel I gefunden.

Alle in der obengenannten Formel auftretenden Alkylgruppen können sowohl geradkettig als auch verzweigt sein.

Wenn in der obengenannten Formel substituierte Phenylgruppen auftreten, so können als Substituenten z.B. C₁-C₄-Alkyl, C₁-C₄-Alkoxy oder Halogen, dabei insbesondere Chlor oder Brom, in Betracht kommen. Die Phenylgruppen weisen dann in der Regel 1 bis 3 Substituenten auf.

Wenn in der obengenannten Formel substituierte C₅-C₇-Cycloalkylgruppen auftreten, so können als Substituenten z.B. C₁-C₄-Alkyl oder C₁-C₄-Aminoalkyl in Betracht kommen. Die Cycloalkylgruppen weisen dann in der Regel 1 bis 3 Alkyl- und/oder eine Aminoalkylgruppe auf.

Wenn in der obengenannten Formel durch Methyl substituiertes Piperidinyl auftritt, so weist es in der Regel 1 bis 4 Methylgruppen auf, wobei substituiertes Piperidin-4-yl bevorzugt ist.

Wenn die Reste R² oder R³ C₁-C₁₃-Alkyl bedeuten, das durch einen 5- bis 7-gliedrigen heterocyclischen Rest, der ein Stickstoffatom und gegebenenfalls ein weiteres Heteroatom, ausgewählt aus der Gruppe, bestehend aus Stickstoff, Sauerstoff und Schwefel, aufweist, substituiert ist, so können als Substituenten gesättigte oder aromatische Reste, wie Pyrrolidinyl, Piperidinyl, Morpholinyl, Piperazinyl, N-(C₁-C₄-Alkyl)piperazinyl, Pyrrolyl, Pyrazolyl, Imidazolyl, Oxazolyl, Isooxazolyl, Thiazolyl oder Isothiazolyl, in Betracht kommen.

Wenn in der obengenannten Formel substituierte Alkylreste auftreten, so sind diese in der Regel ein- oder zweifach substituiert.

Reste R¹, R² und R³ sind z.B. Methyl, Ethyl, Propyl, Isopropyl, Butyl, Isobutyl, sec-Butyl, tert-Butyl, Pentyl, Isopentyl, Neopentyl, tert-Pentyl, Hexyl, 2-Methylpentyl, Heptyl, Octyl, 2-Ethylhexyl, Isooctyl, Nonyl, Isononyl, Decyl, Isodecyl, Undecyl, Dodecyl, Tridecyl, 3,5,5,7-Tetramethylnonyl, Isotridecyl (die obigen Bezeichnungen Isooctyl, Isononyl, Isodecyl und Isotridecyl sind Trivialbezeichnungen und stammen von den nach der Oxosynthese erhaltenen Alkoholen - vgl. dazu Ullmanns Encyklopädie der technischen Chemie, 4. Auflage, Band 7, Seiten 215 bis 217, sowie Band 11, Seiten 435 und 436), Benzyl oder 1- oder 2-Phenylethyl.

Reste R¹ sind weiterhin z.B. Phenyl, 2-, 3- oder 4-Methylphenyl, 2,4-Dimethylphenyl, 2-, 3- oder 4-Methoxyphenyl, 2,4-Dimethoxyphenyl, 2-, 3- oder 4-Chlorphenyl, oder 2,4-Dichlorphenyl.

Reste R¹ sind weiterhin z.B. Formyl, Acetyl, Propionyl, Butyryl, Isobutyryl, Pentanoyl, Hexanoyl, Heptanoyl, Octanoyl, 2-Ethylhexanoyl, Benzoyl, 2-, 3- oder 4-Methylbenzoyl, 2,4-Dimethylbenzoyl, 2-, 3- oder 4-Methoxybenzoyl, 2,4-Dimethoxybenzoyl, 2-, 3- oder 4-Chlorbenzoyl oder 2,4-Dichlorbenzoyl.

Reste R² und R³ sind weiterhin z.B. 2-Aminoethyl, 2- oder 3-Aminopropyl, 2- oder 4-Aminobutyl, 5-Aminopentyl, 6-Aminohexyl, 7-Aminoheptyl, 8-Aminooctyl, 3-Aza-3-methylbutyl, 4-Aza-4-methylpentyl, 3-Aza-3-ethylpentyl, 4-Aza-4-ethylhexyl, 5-Amino-3-azapentyl, 6-Amino-3-azahexyl, 6-Amino-4-azahexyl, 7-Amino-4-azaheptyl, 8-Amino-3,6-diazaoctyl, 3-Aminoprop-2-yl, 8-Amino-4-(2-aminoethyl) octyl, 2-(Pyrrolidin-1-yl)ethyl, 2- oder 3-(Pyrrolidin-1-yl)propyl, 2-(Piperidin-1-yl)ethyl, 2- oder 3-(Piperidin-1-yl)propyl, 2-(Morpholin-4-yl)ethyl, 2- oder 3-(Morpholin-4-yl)propyl, 2-(Piperazin-1-yl)ethyl, 2- oder 3-(Piperazin-1-yl)propyl, 2-(4-Methylpiperazin-1-yl)ethyl, 2- oder 3-(4-Methylpiperazin-1-yl)propyl, 2-(Imidazol-1-yl)ethyl, 2- oder 3-(Imidazol-1-yl)propyl, Cyclopentyl, Cyclohexyl, Cycloheptyl, 3-Aminomethyl-3,5,5-trimethylcyclohexyl oder 2,2,6,6-Tetramethylpiperidin-4-yl.

Bevorzugt sind Sulfonamide der Formel I in der R¹ C₁-C₄-Alkyl, Phenyl, C₂-C₄-Alkanoyl oder Benzoyl bedeutet.

Bevorzugt sind weiterhin Sulfonamide der Formel I, in der
- R²: Wasserstoff und
- R³: C₁-C₁₃-Alkyl, das durch 1 oder 2 Imino- oder C₁-C₄-Alkyliminogruppen unterbrochen ist und/oder durch Amino oder einen 5- oder 6-gliedrigen gesättigten oder aromatischen heterocyclischen Rest, der ein oder zwei Stickstoffatome aufweist, substituiert ist,
bedeuten.

Besonders bevorzugt sind Sulfonamide der Formel I, in der R¹ Acetyl bedeutet.

Die neuen Sulfonamide der Formel I können nach an sich bekannten Methoden erhalten werden. Beispielsweise kann man ein Sulfonsäurehalogenid der Formel II 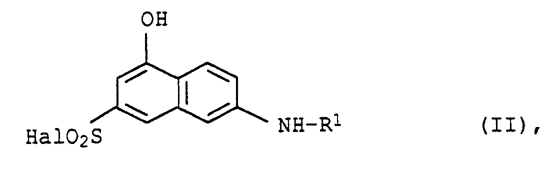 in der Hal Halogen, insbesondere Chlor, bedeutet und R¹ die obengenannte Bedeutung besitzt, mit einem Amin der Formel III 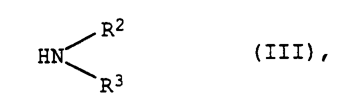 in der R² und R³ jeweils die obengenannte Bedeutung besitzen, umsetzen.

Die Herstellung der Sulfonsäurehalogenide der Formel II kann z.B. gemäß den in Dyes and Pigments, Band 14, Seiten 35 bis 48, 1990, beschriebenen Methoden erfolgen.

Die erfindungsgemäßen Sulfonsäureamide der Formel I sind wertvolle Zwischenprodukte für die Herstellung von Farbstoffen.

Die folgenden Beispiele sollen die Erfindung näher erläutern.

### Beispiel 1

In 30 g Octylamin wurden 6,5 g N-Acetyl-I-Säurechlorid eingetragen und 60 Stunden bei Raumtemperatur gerührt. Die resultierende Lösung wurde anschließend zweimal mit 200 ml Petrolether ausgerührt. Nach dem Abdekantieren des Lösungsmittels erhielt man ein Öl, aus dem durch Zugabe von Wasser und Eisessig 7 g (81 %) Sulfonamid der Formel 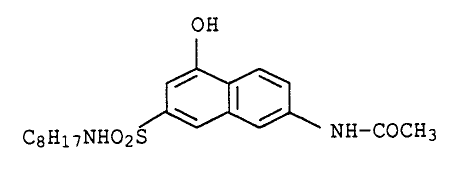 auskristallisierten.

In analoger Weise werden die in der folgenden Tabelle als Beispiel 6, 7 und 8 aufgeführten Verbindungen erhalten.

### Beispiel 2

30 g 1,2-Diaminopropan wurden bei 20°C mit 7 g N-Acetyl-I-Säurechlorid versetzt und 60 Stunden bei Raumtemperatur und weitere 2 Stunden bei 80°C gerührt.

Das resultierende Öl der Formel 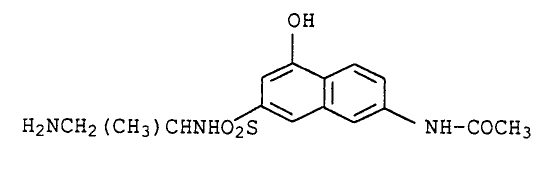 kann direkt in weitere Reaktionen eingesetzt werden.

In analoger Weise werden die in der folgenden Tabelle als Beispiele 3, 4, 5, 9 und 10 aufgeführten Verbindungen erhalten.

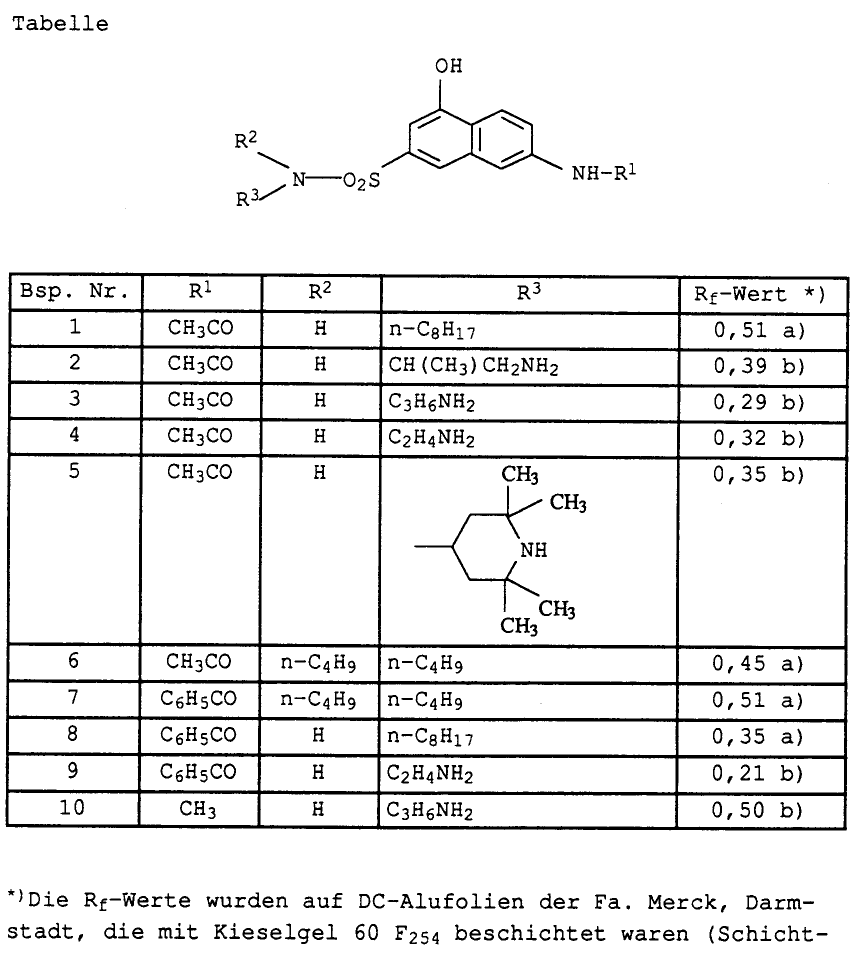

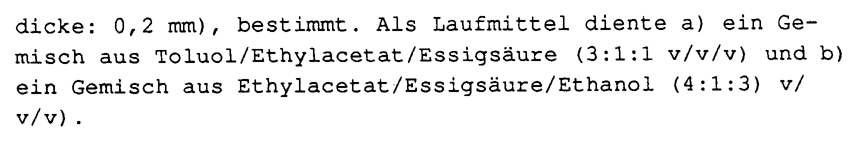

## Patentansprüche

1. Sulfonamide der Formel I 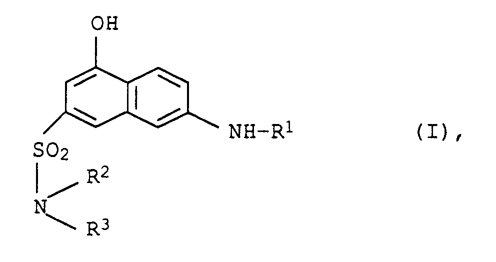 in der
R¹ C₁-C₁₃-Alkyl, gegebenenfalls substituiertes Phenyl, C₁-C₈-Alkanoyl oder gegebenenfalls substituiertes Benzoyl und
R² und R³ gleich oder verschieden sind und unabhängig voneinander jeweils C₁-C₁₃-Alkyl, das gegebenenfalls durch 1 bis 4 Imino- oder C₁-C₄-Alkyliminogruppen unterbrochen ist und durch Amino, einen 5- oder 6-gliedrigen heterocyclischen Rest, der ein Stickstoffatom und gegebenenfalls ein weiteres Heteroatom, ausgewählt aus der Gruppe, bestehend aus Stickstoff, Sauerstoff und Schwefel, aufweist, oder gegebenenfalls substituiertes C₅-C₇-Cycloalkyl substituiert sein kann, gegebenenfalls substituiertes C₅-C₇-Cycloalkyl oder gegebenenfalls durch Methyl substituiertes Piperidinyl oder R² auch Wasserstoff bedeuten.

2. Sulfonamide nach Anspruch 1, dadurch gekennzeichnet, daß R¹ C₁-C₄-Alkyl, Phenyl, C₂-C₄-Alkanoyl oder Benzoyl bedeutet.

3. Sulfonamide nach Anspruch 1, dadurch gekennzeichnet, daß
R² Wasserstoff und
R³ C₁-C₁₃-Alkyl, das durch 1 oder 2 Imino- oder C₁-C₄-Alkyliminogruppen unterbrochen ist und/oder durch Amino oder einen 5- oder 6-gliedrigen gesättigten oder aromatischen heterocyclischen Rest, der ein oder zwei Stickstoffatome aufweist, substituiert ist,
bedeuten.

## Claims

1. A sulfonamide of the formula I 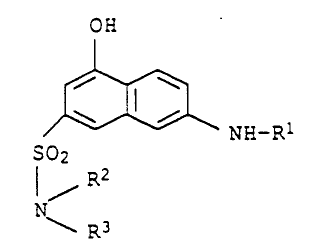 where
R¹ is C₁-C₁₃-alkyl, substituted or unsubstituted phenyl, C₁-C₈-alkanoyl or substituted or unsubstituted benzoyl, and
R² and R³ are identical or different and each is independently of the other C₁-C₁₃-alkyl, optionally interrupted by from 1 to 4 imino or C₁-C₄-alkylimino groups and optionally substituted by amino, by a 5- or 6-membered heterocyclic radical containing a nitrogen atom and optionally a further hetero atom selected from the group consisting of nitrogen, oxygen and sulfur, or by substituted or unsubstituted C₅-C₇-cycloalkyl, substituted or unsubstituted C₅-C₇-cycloalkyl or unsubstituted or methyl-substituted piperidinyl, or R² may else be hydrogen.

2. A sulfonamide as claimed in claim 1, wherein R¹ is C₁-C₄-alkyl, phenyl, C₂-C₄-alkanoyl or benzoyl.

3. A sulfonamide as claimed in claim 1, wherein
R² is hydrogen and
R³ is C₁-C₁₃-alkyl which is interrupted by 1 or 2 imino or C₁-C₄-alkylamino groups and/or is substituted by amino or by a 5- or 6- membered saturated or by a romatic heterocyclic radical containing one or two nitrogen atoms.

## Revendications

1. Sulfonamides de formule I 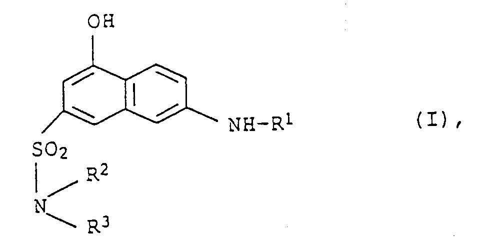 dans laquelle
R¹ représente un groupement alkyle en C₁-C₁₃, un groupement phényle éventuellement substitué, un groupement alcanoyle en C₁-C₈ ou un groupement benzoyle éventuellement substitué et
R² et R³ sont identiques ou différents et représentent chacun indépendamment l'un de l'autre un groupement alkyle en C₁-C₁₃, qui est éventuellement interrompu par 1 à 4 groupements imino ou alkylimino en C₁-C₄ et qui peut être substitué par un groupement amino, un reste hétérocyclique à 5 ou 6 maillons, qui contient un atome d'azote et éventuellement un autre hétéroatome choisi dans le groupe constitué de l'azote, l'oxygène et le soufre, ou par un groupement cycloalkyle en C₅-C₇ éventuellement substitué, un groupement cycloalkyle en C₅-C₇ éventuellement substitué ou un groupement pipéridinyle éventuellement substitué par un groupement méthyle, ou R² peut aussi représenter un atome d'hydrogène.

2. Sulfonamides selon la revendication 1, caractérisé en ce que R¹ représente un groupement alkyle en C₁-C₄, phényle, alcanoyle en C₂-C₄, ou benzoyle.

3. Sulfonamides selon la revendication 1, caractérisé en ce que
R² représente un atome d'hydrogène et
R³ représente un groupement alkyle en C₁-C₁₃, qui est interrompu par 1 ou 2 groupements imino ou alkylimino en C₁-C₄ et/ou substitué par un groupement amino ou par un reste hétérocyclique aromatique ou saturé a 5 ou 6 maillons, qui contient un ou deux atomes d'azote.
